# EUROPEAN PATENT APPLICATION

(11) **EP 1 516 636 A2**
(43) Date of publication of application: **23.03.2005**
(21) Application number: 04077581.9
(22) Date of filing: 17.09.2004
(51) Int. Cl.: A61L 27/18

(54) **Use of a curable elastomer-precursor for a medical treatment**

(30) Priority: 19.09.2003 US 504349 P; 15.03.2004 US 553100 P; 08.04.2004 US 560393 P
(71) Applicant: Broockeville Corporation N.V., Willemstad, Curaçao (NL)
(72) Inventor: de Vries, Jan Albert, 7021 HH Zelhem (NL)
(74) Representative: Volmer, Johannes Cornelis

(57) **Abstract**

Urinary incontinence such as stress incontinence, is alleviated in a mammal, in particular a human being, especially a woman, by correcting the internal urethral orifice and the urethra with an elastic form stable material. Such a material preferably a curable elastomer-precursor composition, is injected in the body tissue surrounding the urethra, thereby correcting internal urethral orifice and the urethra substantially to its original shape, allowing the sphincter to act in a controllable way. In a similar way disorders of the vertebrae of a living creature, for example derived from osteoporosis, can be treated by injection of such an elastic form stable material into the respective vertebral body. This material is also suitable for treating gastroesophageal reflux disease in a mammal such as a human being, by injection thereof into the body tissue in the vicinity of or surrounding the lower esophagus.

## Description

### BACKGROUND OF FIRST ASPECT OF THE INVENTION

### FIELD OF THE FIRST ASPECT OF THE INVENTION

According to a first aspect, the present invention relates to the manufacture of a composition for treatment of incontinence, in particular stress urinary incontinence, in mammals, in particular human beings, especially females. More specifically this aspect of the invention relates to the use of a certain composition in such a manufacturing method.

### DESCRIPTION OF RELATED ART OF THE FIRST ASPECT OF THE INVENTION

Stress incontinence is a phenomenon which frequently occurs in human beings, in particular elder human beings. This uncontrolled and unvoluntary leakage of urine is related to physical activities which strain the abdominal muscles such as coughing, sneezing, lifting of heavy objects, climbing of stairs, etc. The sphincter cannot sufficiently contract in order to resist the increased tension in the abdomen including enlarged tension on the bladder, and undesirable loss of urine is the result. This condition is more common in the female population and is usually associated with weakened pelvic floor muscles related to multiple births and menopause. Also a deficiency of certain female hormones (estrogens) during the menopause can effect the sphincter of the bladder. In the male population, it may be related to certain surgical procedures. Obesity, e.g. derived from diabetus mellitus is another possible cause. Although stress incontinence is usually a periodically occuring phenomenon, the leaking can worsen and become constant.

In order to treat this kind of incontinence various therapies exist. Examples thereof comprise physiotherapy in order to strengthen the pelvic floor muscles by exercise and training. Therapy with hormones is another example. Lowering of the uterus can be counteracted by a pessarium. However, if these therapies do not have the desired effect, surgery is a further option. One of the surgical options is the application of a tension free vaginal tape. This tape is positioned at a position under the urethra, and supports this during activities such as coughing, laughing and lifting. Another surgical method comprises so-called colposuspensions, e.g. according to Burch, wherein the mouth of the urethra is raised and suspended to the inner side of the pubic bone. Other physicians are also using injections of collagen or silicone particles in the wall of the urethra to bulk up the urethra and try to limit leakage that way. Thereby the cross dimension of the urethra is reduced, as a result of which the urine can flow less easily from the bladder. However, collagen is resorbable by the body, as a result of which it is apt to reduce its action. Particles, such as the silicone particles are apt to migrate in the body. Thus their position is not maintained, as a result of which the effect is not ensured, and even reduced in time.

Although some of the treatments mentioned above have shown to be succesfull at least initially at the start of the treatment, the success rate is not easy to predict. Moreover, as the general bodily condition of a patient suffering from incontinence may change over the years, the effectiveness of a certain therapy may become less. In particular injection of particulate material has not been attractive to patients and therefore has not been accepted broadly. Moreover, it is known that the success rate of this kind of treatment is far less compared to the other techniques mentioned above.

As a consequence, there is still a continued need for alternative methods in addition to the existing methods of counteracting stress incontinence, in particular methods which are easy to perform.

There is also a continued need for materials which are effective in counteracting stress incontinence when applied to a mammal, such as a human being.

### SUMMARY OF THE FIRST ASPECT OF THE INVENTION

This aspect of the present invention provides for the use of a curable elastomer-precursor composition in the manufacture of a composition for treatment of incontinence in a mammal, in particular human beings, especially women. This treatment composition is to be used in a method of treating incontinence in a mammal including human beings, in particular women, the method comprising a step of correcting the shape of the internal urethral orifice and the urethra with an elastic form stable material, derived from a curable elastomer-precursor composition. According to the invention, human beings in particular elder women suffering from stress incontinence, are treated by remodelling of the internal urethral orifice (the transition from the bladder to the urethra) and urethra into a shape wherein the effect of the force applied by the sphincter to the urethra and bladder in order to keep it closed, is enhanced. This favourable shape of the urethra is permanently induced by the form stable material resulting after curing of the curable elastomer composition comprised in the treatment composition made according to the invention. After curing, this material, being non-resorbable, is a solid mass compared to particulate material and cannot migrate through the body, but holds its initial position where it is applied. As a result it has been found that substantially normal control over the sphincter muscles is re-established, and the incontinence problems that had previously existed, are substantially eliminated. Simultaneously the resulting form stable material itself is flexible enough to adapt to body movements, so that a patient treated in accordance with the invention does not experience any discomfort. Preferably the elastic form stable material is derived from curing an elastomer-precursor composition in situ. That is to say, the elastomer precursor is prepared in advance and then applied to the appropriate positions by suitable equipment. At these positions the curing of the material thus applied is completed. Surprisingly, it was found that a form stable material made from an elastomeric composition not only is more convenient to the patient treated, but also allows to remodel the internal urethral orifice and the urethra into its original shape such that forces exerted by the sphincter are sufficient to prevent leakage of urine.

The invention also relates to a method of preparing a composition for the treatment of incontinence in a mammal, in particular human beings, especially women, which composition comprises an elastic form stable material, preferably a curable elastomer-precursor composition. Said treatment of incontinence is the shape correction of the urethra and internal urethral orifice.

The invention also relates to an annular support, applied in a human body, obtained after curing of a curable elastomer-precursor composition.

### DESCRIPTION OF THE PREFERRED EMBODIMENTS OF THE FIRST ASPECT OF THE INVENTION

Preferred embodiments of the first aspect of the invention are defined in the subclaims.

Preferably, the abovementioned correction step comprises injecting a curable elastomer-precursor composition in the body tissue surrounding the urethra. Contrary to the collagen and silicone therapies according to the state of the art, wherein the composition is applied in the wall of the urethra, the correcting composition according to the invention is injected through the wall of the urethra in the tissue directly adjacent to the urethra. Preferably the composition is injected in at least three peripheral positions in order to allow said correction. In this way, the injected composition peripherally surrounds the urethra. More preferably several injections are carried out in such a way that the cured composition completely surrounds the urethra as an annular array.

Commercially available medical grade silicone elastomers are preferred materials for use as polymer precursor in the remodelling composition. A more preferred material is poly(dimethyl siloxane) such as hydroxyl-end-blocked poly(dimethyl siloxane). Such silicone elastomers of medical grade as pourable, two-component silicone are available from e.g. NuSil Technology. These silicone elastomers are fast curing materials. For these types of elastomers propyl orthosilicate is a useful cross-linking agent. Fillers and diluents (medicinal fluids such as known under the trade name Dimeticonum) in order to reduce viscosity may be added as needed. An initiator like tin (II) octoate initiates the polymerisation reaction with splitting of propanol. The reaction proceeds without the generation of sensible heat. Silphenylene polymer can be used in a similar way. In order to be able to trace by X-ray monitoring the position of the cured applied composition, in a preferred embodiment the composition comprises a radiopaque material, such as silver powder, barium sulfate or bismuth trioxide.

Here it is noted that a composition as explained above is known per se as a material suitable for the non-surgical, reversible sterilization of females. In this known sterilization method the composition is injected in the oviduct portion adjacent the uterus, where it forms in situ a block or plug in the oviduct, thereby preventing the passage of ovum from the ovaries to the uterus and sperm from entering the oviduct and thus conception. See e.g. US-A-4,245,623.

A preferred composition for use in the method according to the invention comprises about 60 -75 % by weight poly(dimethyl siloxane), about 2-5 % cross-linking agent, a diluent in the range of 10-20 % and about 10-20 % radiopaque powder, based on the weight of the entire composition. An even more preferred composition comprises about 68 wt.% poly(dimethyl siloxane), about 4 % cross-linking agent, about 13 % dimeticonum and about 16 % silver powder.

Prior to application in the body, the composition is advantageously prepared in advance in a mixing-dispensing device. Such a device, wherein the function of mixing the components is combined with the function of dispensing the thus prepared mixture is known per se, e.g. from the above US patent, the content of which is incorporated in its entirety by reference.

As already indicated above, the invention also relates to a method of preparing a composition for the treatment of incontinence, in particular stress incontinence, in mammals in particular human beings, especially women, which composition comprises an elastic form stable composition, preferably a curable elastomer-precursor composition. The above-mentioned preferred features are similarly applicable to the preparation method according to the invention. Advantageously the correcting composition is packaged as a kit of parts, comprising at least a first container filled with an elastomer precursor and optionally a diluent, and a second container filled with a cross-linking agent for this elastomer precursor. More preferably, the composition is packaged in a mixing-dispensing device, comprising such containers and a temporarily seal between the containers, wherein one of these containers is provided with a stirrer which can be operated manually or powered by an external source. An example of such a device is also known from the above-mentioned US patent. Devices of this type can be used for injecting the thus prepared precursor composition by connecting a suitable flexible tube to the container acting as a mixing chamber and providing an appropriate needle at the other end of the tube.

In the invention a flowable composition is prepared from the various components, preferably in a combined mixing-dispensing device as explained above, and then immediately used. The patient is prepared for the treatment according to standard medical procedures. A catheter is brought into the urethra until the injection needle reaches the bladder, e.g. indicated by the presence of a droplet of urine, and then retracted over a predetermined distance, e.g. in the range of 1-2 cm. At the position thus reached the wall of the urethra is punctured by the injection needle, and the composition is forced from the respective container via a suitable flexible tube to the needle and deposited directly adjacent this wall in the respective tissue. In order to more accurately position the deposit of the composition, preferably the needle is not open at the tip but has one or more exits at the side face directly adjacent to the tip. Use of this modified needle allows to position the composition more easily adjacent the urethra wall. Thereafter the needle is retracted and the composition is allowed to cure in situ. As already mentioned preferably the composition is applied at multiple locations surrounding the urethra. In view thereof the above actions can be repeated as needed.

### BACKGROUND OF SECOND ASPECT OF THE INVENTION

### FIELD OF THE SECOND ASPECT OF THE INVENTION

According to a second aspect, the present invention relates to the manufacture of a composition for treatment of a disordered vertebral body in a living creature, in particular a human being, by vertebroplasty. More specifically this aspect relates to the use of a certain composition suitable for use in such a manufacturing method.

### DESCRIPTION OF RELATED ART OF THE SECOND ASPECT OF THE INVENTION

Percutaneous vertebroplasty comprises the injection of bone cement into a vertebral body to be treated via a percutaneous route, usually under X-ray guidance, such as lateral projection fluoroscopy. The bone cement is injected as a paste or semi-liquid from a suitable cement gun or injection system via a needle, that has been passed into the vertebral body. Vertebroplasty is usually applied to patients that suffer from osteoporotic fractures, malignant metastatic disease and benign tumors of the bone. The bone cement once injected should provide a reinforcement to and improved compressive strength of the vertebral body. In addition to these reinforcing and strengthening properties it is desirable that the starting bone cement composition is of a viscosity that allows it to flow into the fracture planes.

At present almost all bone cement is based on polymethyl methacrylate. This type of bone cement is prepared from a mixture of methyl methacrylate powder and a suitable radiopaque agent (opacifier) to which a liquid monomer is added. Then the resulting cement should be injected within a limited period of time, e.g. 5 to 10 minutes, before the cement starts to thicken and becomes unworkable. While the cement is being injected, continuous X-ray imaging is performed in order to watch carefully whether the cement is deposited in the appropriate position and proportion, typically some cubic centimetres, within the vertebral body. The cement thus injected is then cured within the vertebral body to a hard material. However, it has appeared that this type of bone cement gives some disadvantages. Due to its hardness and strength the cured material can easily distort the brittle bone material of the vertebral body into which it has been injected. Furthermore there is a risk of leakage of material from the vertebral body, thereby cutting through tissue and nerves.

Thus there is a need for a vertebroplasty material, which does not present the above disadvantages, or at least to a lesser extent.

### SUMMARY OF THE SECOND ASPECT OF THE INVENTION

The second aspect of the present invention provides for the use of a curable elastomer-precursor composition in the manufacture of a bone cement composition for treating a disordered vertebral body in a living creature, in particular a human being. This composition is to be used in a vertebroplasty method of treating a disordered vertebral body in a living creature, in particular a human being, the method comprising the step of injecting a curable bone cement composition in said vertebral body, wherein said curable bone cement comprises a curable elastomer-precursor composition. After curing, an elastic form stable material is obtained.

According to the invention, a creature such as a human being in need of a vertebroplasty treatment e.g. derived from osteoporosis, is treated by reinforcing and strengthening the respective vertebral body or bodies. To this purpose a bone cement is injected into the selected vertebral body, wherein the bone cement is a curable elastomer-precursor composition, resulting into an elastic form stable material. Due to its properties with respect to elasticity and dimensional stability the forces exerted on the brittle vertebral body upon and after injecting the bone cement composition are smaller compared to the state of the art, thereby reducing the risk of breaking or disrupting the body thus treated. After curing this material being non-resorbable, is a solid but still flexible mass that cannot migrate through the body, but holds its initial position where it is applied.

Typically the vertebroplasty method using the bone cement composition according to the invention comprises the following steps. The patient is examined usually by CT and/or MR imaging in order to provide the data upon which a decision about the vertebral body to be treated is made. The patient to be treated is positioned in a prone position, and the skin covering the vertebral body is prepared. The patient is anaesthetized, usually locally by injecting a suitable anaesthetic into the skin underlying fat and into the periosteum of the pedicle to be entered. Next a skin incision is made using a scalpel or other surgical instrument. A needle having a suitable gauge is selected and passed down the pedicle into the respective vertebral body. If desired, any unwanted leakage can be detected by injecting a suitable X-ray contrast liquid into the vertebral body and performing a vertebrogram. Meanwhile the bone cement is prepared and is then injected, while monitoring the position of the needle, the position and amount of injected cement, e.g. by lateral X-ray projection fluoroscopy imaging. For that purpose a suitable opacifier is added to the bone cement. The injection is stopped when the filling of the vertebral body has been completed to the desired extent. If the cement starts to flow into unwanted locations, injection will also be interrupted.

Preferably the elastic form stable material comprises an elastomer-precursor composition which is cured in situ. That is to say, the elastomer precursor is prepared in advance and then applied to the appropriate vertebral body by suitable equipment.

The invention also relates to a method of preparing a composition for treating a disordered vertebral body in a living creature, in particular a human being, comprising an elastic form stable material. Usually this manufacturing method comprises the steps of providing the starting materials of the elastic form stable materials in the appropriate ratio into separate containers (or separated chambers that may be present in one mixing/dispensing device), and packaging thereof.

### DESCRIPTION OF THE PREFERRED EMBODIMENTS OF THE SECOND ASPECT

Preferred embodiments of the second aspect of the invention are defined in the subclaims.

According to a preferred embodiment, the vertebroplasty composition comprises a curable elastomer-precursor composition. Commercially available medical grade silicone elastomers are preferred materials for use as polymer precursor in this composition. A more preferred material is poly(dimethyl siloxane) such as hydroxyl-end-blocked poly(dimethyl siloxane). Such silicone elastomers of medical grade as pourable, two-component silicone are available from e.g. NuSil Technology. These silicone elastomers are fast curing materials. For these types of elastomers propyl orthosilicate is a useful cross-linking agent. Fillers and diluents (medicinal fluids such as known under the trade name Dimeticonum) in order to reduce viscosity may be added as needed. It is preferred that a well flowable composition is used, so that even small hair cracks are filled with the composition. An initiator like tin (II) octoate initiates the polymerisation reaction with splitting of propanol. The reaction proceeds without the generation of sensible heat, which is also benificial to the patient treated. Silphenylene polymer can be used in a similar way. In order to be able to trace by X-ray monitoring the position of the curing/cured applied composition, in a preferred embodiment the composition comprises a radiopaque material, such as silver powder, barium sulfate or bismuth trioxide. Silver powder is the most preferred radiopaque material, because compared to the other examples a smaller proportion is needed in the composition.

Here it is noted that a composition as explained above is known per se as a material suitable for the non-surgical, reversible sterilization of females. In this known sterilization method the composition is injected in the oviduct portion adjacent the uterus, where it forms in situ a block or plug in the oviduct, thereby preventing the passage of ovum from the ovaries to the uterus and sperm from entering the oviduct and thus conception. See e.g. US-A-4,245,623.

A preferred bone cement composition comprises about 60 -75 % by weight poly(dimethyl siloxane), about 2-5 % cross-linking agent, a diluent in the range of 10-20 % and about 10-20 % radiopaque powder, based upon the weight of the entire composition. An even more preferred composition comprises about 68 wt.% poly(dimethyl siloxane), about 4 % cross-linking agent, about 13 % dimeticonum and about 16 % silver powder.

In the vertebroplasty method using the composition according to the invention the composition is advantageously prepared in advance in a mixing-dispensing device. Such a device, wherein the function of mixing the components is combined with the function of dispensing the thus prepared mixture, is known per se, e.g. from the above US patent, the content of which is incorporated in its entirety by reference.

As already indicated above, the invention also relates to a method of preparing an injection composition for injection into a disordered vertebral body of a living creature, in particular a human being, said composition comprising an elastic form stable material.

The above-mentioned preferred features mentioned above are similarly applicable to the preparation method according to the invention. Advantageously the injection composition is packaged as a kit of parts, comprising at least a first container filled with an elastomer precursor and optionally a diluent, and a second container filled with a cross-linking agent for this elastomer precursor. More preferably, the composition is packaged in a mixing-dispensing device, comprising such containers and a temporarily seal between the containers, wherein one of these containers is provided with a stirrer which can be operated manually or powered by an external source. An example of such a device is also known from the above-mentioned US patent. Devices of this type can be used for injecting the thus prepared precursor composition by connecting a suitable flexible tube to the container acting as a mixing chamber and providing an appropriate needle at the other end of the tube.

In the invention a flowable composition is prepared from the various components, preferably in a combined mixing-dispensing device as explained above, and then immediately used. In order to more accurately position the deposit of the composition, preferably the needle, in addition to an open tip, has one or more exits at the side face directly adjacent to the tip. Use of this modified needle allows to position the composition more easily at the required positions. Thereafter the needle is retracted and the composition is allowed to cure in situ. If necessary, the above actions can be repeated as needed.

### BACKGROUND OF THE THIRD ASPECT OF THE INVENTION

### FIELD OF THE THIRD ASPECT OF THE INVENTION

According to a third aspect, the present invention relates to the manufacture of a composition for treatment of gastroesophageal reflux disease (abbreviated to GERD) in a mammal, in particular a human being. In particular, this aspect relates to the use of a certain composition in such a manufacturing method.

### DESCRIPTION OF RELATED ART OF THE THIRD ASPECT

Gastroesophageal reflux disease relates to the repeated backup of food and digestive fluid from the stomach into the esophagus. Symptoms thereof comprise heartburn and acid indigestion. GERD is caused by malfunctioning of the lower esophageal sphincter, a small ring of muscles. GERD, if not treated, can lead to serious disorders or complications, such as bleeding and narrowing of the esophagus. GERD can be treated in a variety of ways ranging from changing lifestyle, treatment by drugs, prothesis and surgery. An alternative to surgery, e.g. known from WO 03/072196 involves modifying the lower esophagus by injecting an emulsion of microparticulate material in a suitable liquid carrier into body tissue sites surrounding the esophagus, in particular the submucosal tissue site adjacent to or in the vicinity of the lower esophageal sphincter (hereinafter abbreviated to LES). The dimensions of the microparticulate material are critical. The particles should be able to pass through a hypodermic needle, while after injection they should substantially remain at the injection site. However, despite the careful selection of suitable dimensions for the microparticulate material, the risk remains that these particles are susceptible to migration.

The object of the invention is to provide materials for use in the treatment of gastroesophageal reflux disease in a mammal, in particular a human being, wherein the risk of migration of microparticulate material is reduced.

### SUMMARY OF THE THIRD ASPECT OF THE INVENTION

This aspect of the invention provides for the use of a curable elastomer-precursor composition in the manufacture of a composition for treatment of gastroesophageal reflux disease in a mammal, in particular a human being.

Use of such a treatment composition for treating GERD comprises injecting a curable elastomer-precursor composition into the body tissue in the vicinity of or surrounding the lower esophagus, and allowing the composition to cure to an elastic form stable material. An elastomer-precursor composition is prepared, and subsequently injected in one or more injection sites adjacent to or in the vicinity of the LES. After injection the composition cures to an elastic form stable material. The cured material, which is a solid mass, holds its initial position, and the risk of migration is significantly reduced. The absence of microparticulates in the material used in the invention also contributes to the reduction of the risk of migration. The form stability of the material ensures that the respective tissue is sufficiently strengthened in order to enhance the appropriate operation of the LES, thereby preventing reflux of the content of the stomach back into the esophagus. Nevertheless, a patient treated according to the invention is still able to vomit naturally, because of the elasticity of the form stable material. Non-elastic material would complicate such vomitting severely. Simultaneously the form stable material itself is flexible enough to adapt to body movements, so that a patient treated according to the invention does not experience any discomfort.

The invention also relates to a method of preparing a composition for the treatment of gastroesophageal reflux disease in a mammal, in particular a human being, comprising a curable elastomer-precursor composition.

The invention also relates to the use of an elastic form stable material, preferably a curable elastomer-precursor composition in the preparation of a treating composition for the treatment of gastroesophageal reflux disease in a mammal, in particular a human being.

### DESCRIPTION OF THE PREFERRED EMBODIMENTS OF THE THIRD ASPECT

Preferred embodiments of the third aspect are defined in the subclaims.

The elastic form stable material comprises an elastomer-precursor composition which is cured in situ. That is to say, the elastomer precursor is prepared in advance and then applied to the appropriate positions by suitable equipment. Preferably the composition is injected in at least three peripheral positions surrounding the LES. More preferably several injections are carried out in such a way that the cured composition completely surrounds the esophagus, in particular at the LES, as an annular support.

Commercially available medical grade silicone elastomers are preferred materials for use as polymer precursor in the remodelling composition. A more preferred material is poly(dimethyl siloxane) such as hydroxyl-end-blocked poly(dimethyl siloxane). Such silicone elastomers of medical grade as pourable, two-component silicone are available from e.g. NuSil Technology. These silicone elastomers are fast curing materials. For these types of elastomers propyl orthosilicate is a useful cross-linking agent. Fillers and diluents (medicinal fluids such as known under the trade name Dimeticonum) in order to reduce viscosity may be added as needed. An initiator like tin (II) octoate or platinum initiates the polymerisation reaction with splitting of propanol. The reaction proceeds without the generation of sensible heat. Silphenylene polymer can be used in a similar way. In order to be able to trace by X-ray monitoring the position of the cured applied composition, in a preferred embodiment the composition comprises a radiopaque material, such as silver powder, barium sulfate or bismuth trioxide. Silver powder is the most preferred opacifier.

Here it is noted that a composition as explained above is known per se as a material suitable for the non-surgical, reversible sterilization of females. In this known sterilization method the composition is injected in the oviduct portion adjacent the uterus, where it forms in situ a block or plug in the oviduct, thereby preventing the passage of ovum from the ovaries to the uterus and sperm from entering the oviduct and thus conception. See e.g. US-A-4,245,623.

A preferred composition comprises about 60 -75 % by weight poly(dimethyl siloxane), about 2-5 % cross-linking agent, a diluent in the range of 10-20 % and about 10-20 % radiopaque powder, based on the weight of the entire composition. An even more preferred composition comprises about 68 wt.% poly(dimethyl siloxane), about 4 % cross-linking agent, about 13 % dimeticonum and about 16 % silver powder

The composition is advantageously prepared in advance in a mixing-dispensing device. Such a device, wherein the function of mixing the components is combined with the function of dispensing the thus prepared mixture is known per se, e.g. from the above US patent, the content of which is incorporated in its entirety by reference.

As already indicated above, the invention also relates to a method of preparing a composition for the treatment of gastroesophageal reflux disease in a mammal, in particular a human being, comprising a curable elastomer-precursor composition.

The above-mentioned preferred features are similarly applicable to the preparation method according to the invention.

Advantageously the composition is packaged as a kit of parts, comprising at least a first container filled with an elastomer precursor and optionally a diluent, and a second container filled with a cross-linking agent for this elastomer precursor. More preferably, the composition is packaged in a mixing-dispensing device, comprising such containers and a temporarily seal between the containers, wherein one of these containers is provided with a stirrer which can be operated manually or powered by an external source. An example of such a device is also known from the above-mentioned US patent. Devices of this type can be used for injecting the thus prepared precursor composition by connecting a suitable flexible tube to the container acting as a mixing chamber and providing an appropriate needle at the other end of the tube.

In the invention a flowable composition is prepared from the various components, preferably in a combined mixing-dispensing device as explained above, and then immediately used. The patient is prepared for the treatment according to standard medical procedures. A catheter is brought via the mouth into the esophagus until the injection needle reaches the vicinity of the LES. At the position thus reached the wall of the esophagus is punctured by the injection needle, and the composition is forced from the respective container via a suitable flexible tube to the needle and deposited directly adjacent this wall in the respective tissue. In order to more accurately position the deposit of the composition, preferably the needle is not open at the tip but has one or more exits at the side face directly adjacent to the tip. Use of this modified needle allows to position the composition more easily adjacent the esophagus wall into the tissue. Thereafter the needle is retracted and the composition is allowed to cure in situ. As already mentioned preferably the composition is applied at multiple locations surrounding the esophagus. In view thereof the above actions can be repeated as needed. Upon multiple injections an annular array, applied in body tissue substantially surrounding the lower esophagus, obtained after curing of a curable elastomer-precursor composition can be obtained.

## Claims

1. Use of a curable elastomer-precursor composition in the manufacture of a composition for treatment of incontinence in a mammal, in particular human beings, especially women.

2. Use according to claim 1, wherein the curable elastomer-precursor composition comprises a silicone elastomer.

3. Use according to claim 2, wherein said silicone elastomer is poly(dimethoxy siloxane).

4. Use according to one of the preceding claims, wherein said composition comprises a silicone elastomer, a cross-linking agent and a diluent.

5. Use according to one of the preceding claims, wherein said composition comprises a radiopaque material.

6. Use according to one of the preceding claims, wherein said composition is packaged as a kit of parts, comprising at least a first container with said silicone elastomer, and a second container with said cross-linking agent.

7. Use according to claim 6, wherein said kit of parts is a mixing-dispensing device, comprising said containers and a temporarily sealing between the containers, wherein one container is provided with a movable stirrer.

8. Use of an elastic form stable material in the manufacture of a composition for treatment of incontinence in a mammal, in particular human beings, especially women.

9. Annular array, applied in body tissue substantially surrounding the urethra, obtained after curing a curable elastomer-precursor composition.

10. Use of a curable elastomer-precursor composition in the manufacture of a bone cement composition for treating a disordered vertebral body in a living creature, in particular a human being.

11. Use according to claim 10, wherein the curable elastomer-precursor composition comprises a silicone elastomer.

12. Use according to claim 11, wherein said silicone elastomer is poly(dimethoxy siloxane).

13. Use according to one of the preceding claims 10-12, wherein said composition comprises a silicone elastomer, a cross-linking agent and a diluent.

14. Use according to one of the preceding claims 10-13, wherein said composition comprises a radiopaque material.

15. Use according to one of the preceding claims 10-14, wherein said composition is packaged as a kit of parts, comprising at least a first container with said silicone elastomer, and a second container with said cross-linking agent.

16. Use according to claim 15, wherein said kit of parts is a mixing-dispensing device, comprising said containers and a temporarily sealing between the containers, wherein one container is provided with a movable stirrer.

17. Use of an elastic form stable material in the manufacture of a composition for treatment of a disordered vertebral body in a living creature, in particular a human being.

18. Use of a curable elastomer-precursor composition in the manufacture of a composition for treating gastroesophageal reflux disease in a mammal, in particular a human being.

19. Use according to claim 18, wherein the curable elastomer-precursor composition comprises a silicone elastomer.

20. Use according to claim 19, wherein said silicone elastomer is poly(dimethoxy siloxane).

21. Use according to one of the preceding claims 18-20, wherein said composition comprises a silicone elastomer, a cross-linking agent and a diluent.

22. Use according to one of the preceding claims 18-21, wherein said composition comprises a radiopaque material.

23. Use according to one of the preceding claims 18-22, wherein said composition is packaged as a kit of parts, comprising at least a first container with said silicone elastomer, and a second container with said cross-linking agent.

24. Use according to claim 23, wherein said kit of parts is a mixing-dispensing device, comprising said containers and a temporarily sealing between the containers, wherein one container is provided with a movable stirrer.

25. Annular array, applied in body tissue substantially surrounding the lower esophagus, obtained after curing of a curable elastomer-precursor composition.

26. Use of an elastic form stable material in the preparation of a treating agent for the treatment of gastroesophageal reflux disease in a mammal, in particular a human being.
